# EUROPEAN PATENT SPECIFICATION

(11) **EP 2 560 647 B1**
(45) Date of publication and mention of the grant of the patent: **13.04.2016**
(21) Application number: 11772507.7
(22) Date of filing: 19.04.2011
(51) Int. Cl.: A61K 31/47, A61K 31/69, A61K 33/24, A61P 35/00

(54) **COMBINATION THERAPY WITH A PROTEASOME INHIBITOR AND A GALLIUM COMPLEX**
KOMBINATIONSTHERAPIE MIT EINEM PROTEASOMHEMMER UND EINEM GALLIUMKOMPLEX
POLYTHÉRAPIE AVEC UN INHIBITEUR DU PROTÉASOME ET UN COMPLEXE DE GALLIUM

(30) Priority: 19.04.2010 US 325571 P
(43) Date of publication of application: 27.02.2013
(73) Proprietor: Niiki Pharma Inc., Hoboken, New Jersey 07030 (US)
(72) Inventor: SHESHBARADARAN, Hooshmand, Hoboken, New Jersey 07030 (US)
(74) Representative: Tostmann, Holger Carl
(86) International application number: PCT/US2011/032937
(87) International publication number: WO 2011/133479

(56) References cited:
- US-A- 5 525 598
- US-A1- 2006 275 308
- MICHAEL A JACUPEC, ET AL.: "INTERACTIONS OF TRIS(8-QUINOLINOLATO)GALIUM (III) (KP46) WITH PLATINUM DRUGS IN OVARIAN AND COLON CARCINOMA CELLS", AMERICAN ASSOCIATION FOR CANCER RESEARCH. PROCEEDINGS OF THE ANNUAL MEETING, vol. 42, 17 February 2001 (2001-02-17), page 425, XP002205843, AMERICAN ASSOCIATION FOR CANCER RESEARCH, US ISSN: 0197-016X
- HOFHEINZ R-D ET AL: "EARLY RESULTS FROM A PHASE I STUDY ON ORALLY ADMINISTERED TRIS(8-QUINOLINOLATOLGALLIUM(III) (FFC11, KP46) IN PATIENTS WITH SOLID TUMORS--A CESAR STUDY (CENTRAL EUROPEAN SOCIETY FOR ANTICANCER DRUG RESEARCH--EWIV)", INTERNATIONAL JOURNAL OF CLINICAL PHARMACOLOGY AND THERAPEUTICS, DUSTRI-VERLAG, vol. 43, no. 12, 1 December 2005 (2005-12-01), pages 590-591, XP009066236, DEISENHOFEN-MUENCHEN, DE ISSN: 0946-1965
- SEIED MOJTABA VALIAHDI ET AL: "The gallium complex KP46 exerts strong activity against primary explanted melanoma cells and induces apoptosis in melanoma cell lines", MELANOMA RESEARCH, vol. 19, no. 5, 1 October 2009 (2009-10-01), pages 283-293, XP055071628, ISSN: 0960-8931, DOI: 10.1097/CMR.0b013e32832b272d
- CHITAMBAR ET AL: "A novel gallium compound synergistically enhances Bortezomib induced apaptosis in mantle cell lymphoma", LEUK RES, vol. 34, no. 7, July 2010 (2010-07), pages 950-953, XP002711467,
- GOJO J ET AL: "150 Induction of endoplasmic reticulum stress by the novel anti-cancer compound KP46 and synergism with proteasome inhibition", EUROPEAN JOURNAL OF CANCER. SUPPLEMENT, vol. 8, no. 7, 1 November 2010 (2010-11-01), page 53, XP027497838, PERGAMON, OXFORD, GB ISSN: 1359-6349, DOI: 10.1016/S1359-6349(10)71855-3 [retrieved on 2010-11-01]
- ALEXANDER V. RUDNEV ET AL.: 'Preclinical characterization of anticancer gallium(III) complexes: Solubility, stability, lipophilicity and binding to serum proteins' JOURNAL OF INORGANIC BIOCHEMISTRY vol. 100, no. 11, 2006, pages 1819 - 1826, XP027900306

## Description

### Cross-Reference to Related U.S. Applications

This application claims the benefit of U.S. Provisional Application No. 61/325,571 filed on April 19, 2010, which is incorporated herein by reference.

### Field of the Invention

The present invention generally relates to pharmaceutical compositions and methods for treating cancer, and particularly to a combination therapy of cancer using a proteasome inhibitor and a gallium complex, as defined in the claims.

### Background of the Invention

Bortezomib developed by Millennium Pharmaceuticals (now part of Eisai Pharmaceuticals) as a proteasome inhibitor has been marketed for treating multiple myeloma. In addition, bortezomib has been shown to be useful in preclinical models and is being tested in clinical trials for various other types of cancer. Other proteasome inhibitors are disclosed in U.S. Pat. Nos. 5,780,454 and 7,442,830; PCT Publication Nos. WO 07/0005991, WO 02/096933, WO 05/016859, WO 05/021558 and WO 06/08660.

Tris(8-quinolinolato)gallium(III) is an organic gallium complex that has been suggested to be useful in certain types of cancer. For example, US Patent No. 7,919,486 discloses and claims the use of tris(8-quinolinolato)gallium(III) and related compounds for the treatment of melanoma.

### Summary of the invention

The present invention relates to the following embodiments:
1. Use of a therapeutically effective amount of a compound of Formula (I) 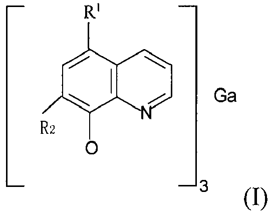 wherein R¹ represents hydrogen, a halogen or a sulfono group SO₃M, in which M is a metal ion, and R² represents hydrogen, or R¹ is Cl and R² is I, or a pharmaceutically acceptable salt thereof for the manufacture of a medicament for use in combination with a proteasome inhibitor for treating a proliferative disorder.
2. A compound of Formula (I) 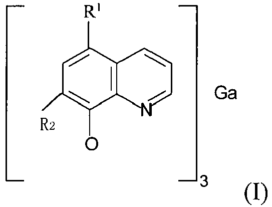 wherein R¹ represents hydrogen, a halogen or a sulfono group SO₃M, in which M is a metal ion, and R² represents hydrogen, or R¹ is Cl and R² is I, or a pharmaceutically acceptable salt thereof for use in treating a proliferative disorder, wherein said compound is to be used in combination with a proteasome inhibitor.
3. A proteasome inhibitor for use in treating a proliferative disorder, wherein said proteasome inhibitor is to be used in combination with a compound of Formula (I) 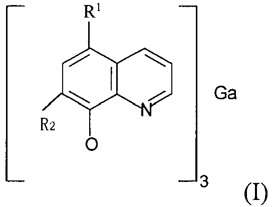 wherein R¹ represents hydrogen, a halogen or a sulfono group SO₃M, in which M is a metal ion, and R² represents hydrogen, or R¹ is Cl and R² is I, or a pharmaceutically acceptable salt thereof.
4. The use of embodiment 1 or the compound for use according to embodiment 2 or the proteasome inhibitor for use according to embodiment 3, wherein said compound of Formula (I) is tris(8-quinolinolato)gallium(III).
5. The use of embodiment 1 or 4 or the compound for use according to embodiment 2 or 4 or the proteasome inhibitor for use according to embodiment 3 or 4, wherein the proteasome inhibitor is bortezomib.
6. The use of embodiment 1 or 4 or the compound for use according to embodiment 2 or 4 or the proteasome inhibitor for use according to embodiment 3 or 4, wherein the proteasome inhibitor is chosen from the group of [(1R)-1-({[(2,3-difluorobenzoyl)amino]acetyl}amino)-3-methylbutyl]boronic acid, [(1R)-1-({[(5-chloro-2-fluorobenzoyl)amino]acetyl}amino)-3-methylbutyl]boronic acid, [(1R)-1-({[(3,5-difluorobenzoyl)amino]acetyl}amino)-3-methylbutyl]boronic acid, [(1R)-1-({[(2,5-difluorobenzoyl)amino]acetyl}amino)-3-methylbutyl]boronic acid, [(1R)-1-({[(2-bromobenzoyl)amino]acetyl}amino)-3-methylbutyl]boronic acid, [(1R)-1-({[(2-fluorobenzoyl)amino]acetyl}amino)-3-methylbutyl]boronic acid, [(1R)-1-({[(2-chloro-5-fluorobenzoyl)amino] acetyl}amino)-3-methylbutyl]boronic acid, [(1R)-1-({[(4-fluorobenzoyl)amino]acetyl}amino)-3-methylbutyl]boronic acid, [(1R)-1-({[(3,4-difluorobenzoyl)amino]acetyl}amino)-3-methylbutyl]boronic acid, [(1R)-1-({[(3-chlorobenzoyl)amino]acetyl}amino)-3-methylbutyl]boronic acid, [(1R)-1-({[(2,5-dichlorobenzoyl)amino]acetyl}amino)-3-methylbutyl]boronic acid, [(1R)-1-({[(3,4-dichlorobenzoyl)amino]acetyl}amino)-3-methylbutyl]boronic acid, [(1R)-1-({[(3-fluorobenzoyl)amino]acetyl}amino)-3-methylbutyl]boronic acid, [(1R)-1-({[(2-chloro-4-fluorobenzoyl)amino]acetyl}amino) -3-methylbutyl]boronic acid, [(1R)-1-({[(2,3-dichlorobenzoyl)amino]acetyl}amino)-3-methylbutyl]boronic acid, [(1R)-1-({[(2-chlorobenzoyl)amino]acetyl}amino)-3-methylbutyl]boronic acid, [(1R)-1-({[(2,4-difluorobenzoyl)amino]acetyl}amino)-3-methylbutyl]boronic acid, [(1R)-1-({[(4-chloro-2-fluorobenzoyl)amino]acetyl}amino)-3-methylbutyl]boronic acid, [(1R)-1-({[(4-chlorobenzoyl)amino]acetyl}amino)-3-methylbutyl]boronic acid, [(1R)-1-({[(2,4-dichlorobenzoyl)amino]acetyl}amino)-3-methylbutyl]boronic acid, and [(1R)-1-({[(3,5-dichlorobenzoyl)amino]acetyl}amino)-3-methylbutyl]boronic acid, and mannitol esters, salts and boronic acid anhydrides thereof.
7. The use of embodiment 1 or 4 or the compound for use according to embodiment 2 or 4 or the proteasome inhibitor for use according to embodiment 3 or 4, wherein the proteasome inhibitor is CEP-18770, carfilzomib or MLN9708.
8. The use of any one of embodiments 1 and 4 to 7 or the compound for use according to any one of embodiments 2 and 4 to 7 or the proteasome inhibitor for use according to any one of embodiments 3 to 7, wherein the proliferative disorder is cancer.
9. The use of any one of embodiments 1 and 4 to 7 or the compound for use according to any one of embodiments 2 and 4 to 7 or the proteasome inhibitor for use according to any one of embodiments 3 to 7, wherein the proliferative disorder is multiple myeloma.
10. The use of any one of embodiments 1 and 4 to 7 or the compound for use according to any one of embodiments 2 and 4 to 7 or the proteasome inhibitor for use according to any one of embodiments 3 to 7, wherein the proliferative disorder is a solid tumor.
11. The use of any one of embodiments 1 and 4 to 7 or the compound for use according to any one of embodiments 2 and 4 to 7 or the proteasome inhibitor for use according to any one of embodiments 3 to 7, wherein the proliferative disorder is a hematological cancer.
12. A kit, comprising in a compartmentalized container:
   a first unit dosage form having a compound of Formula (I) 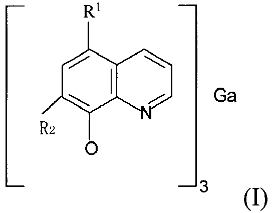 wherein R¹ represents hydrogen, a halogen or a sulfono group SO₃M, in which M is a metal ion, and R² represents hydrogen, or R¹ is Cl and R² is I, or a pharmaceutically acceptable salt thereof; and
      a second unit dosage form having a proteasome inhibitor.
13. The kit of embodiment 12, wherein the compound of Formula (I) is tris(8-quinolinolato)gallium(III).
14. A pharmaceutical composition, comprising a therapeutically effective amount of a compound of Formula (I) 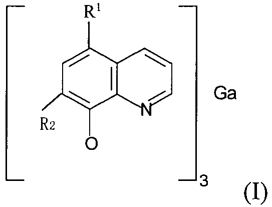 wherein R¹ represents hydrogen, a halogen or a sulfono group SO₃M, in which M is a metal ion, and R² represents hydrogen, or R¹ is Cl and R² is I, or a pharmaceutically acceptable salt thereof and a proteasome inhibitor.
15. The pharmaceutical composition of embodiment 14, wherein said compound of Formula(I) is tris(8-quinolinolato)gallium(III).

It has been surprisingly discovered that the combined use of a compound of Fomrula (I) below, particularly tris(8-quinolinolato)gallium(III), and a proteasome inhibitor such as bortezomib can create unexpected synergies in killing tumor cells and therefore treating cancers. Accordingly, in a first aspect, the present invention provides a compound of Formula (I) for use in a method of treating cancer in a patient in need of such treatment comprising administering, simultaneously or sequentially, to the patient a therapeutically effective amount of the compound of Formula (I) and a proteasome inhibitor such as bortezomib.

In accordance with a second aspect, a pharmaceutical composition is provided comprising a therapeutically effective amount of a compound of Formula (I) below, particularly tris(8-quinolinolato)gallium(III), and a proteasome inhibitor such as bortezomib.

In accordance with another aspect of the present invention, a kit is provided comprising in a compartmentalized container a first unit dosage form having a compound of Formula (I) below, e.g., tris(8-quinolinolato)gallium(III), and a second unit dosage form having a proteasome inhibitor such as bortezomib. Optionally, instructions on how to use the kit are included in the kit.

The foregoing and other advantages and features of the invention, and the manner in which the same are accomplished, will become more readily apparent upon consideration of the following detailed description of the invention taken in conjunction with the accompanying examples, which illustrate preferred and exemplary embodiments.

### Brief Description of the Drawings

**Figure 1** is a conservative isobologram showing the synergism created by the combination of tris(8-quinolinolato)gallium(III) and bortezomib in ARH-77 cells;
**Figure 2** is a graph showing synergism between tris(8-quinolinolato)gallium(III) and bortezomib in A427 cells;
**Figure 3** is a graph showing synergism between tris(8-quinolinolato)gallium(III) and bortezomib in A549 cells.

### Detailed Description of the Invention

The present invention provides a treatment of cancer by a combination therapy which comprises treating a cancer patient in need of treatment with a therapeutically effective amount of (1) a compound of Formula (I) 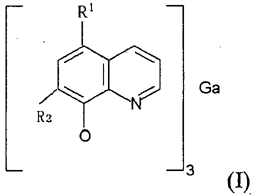 wherein R¹ represents hydrogen, a halogen or a sulfono group SO₃M, in which M is a metal ion, and R² represents hydrogen, or R¹ is Cl and R² is I, or a pharmaceutically acceptable salt thereof; and (2) a proteasome inhibitor.

As used herein, the phrase "treating ... with ..." means administering a compound to the patient or causing the formation of a compound inside the patient. As used herein, the term "pharmaceutically acceptable salts" refers to the relatively non-toxic, organic or inorganic salts of the active compounds, including inorganic or organic acid addition salts of the compound.

In one embodiment, the treatment of cancer comprises (1) administering to a cancer patient in need of treatment a therapeutically effective amount of a compound of 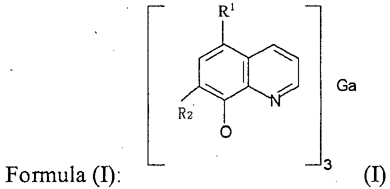 wherein R¹ represents hydrogen, a halogen or a sulfono group SO₃M, in which M is a metal ion, and R² represents hydrogen, or R¹ is Cl and R² is I, or a pharmaceutically acceptable salt thereof; and (2) administering to said cancer patient a therapeutically effective amount of a proteasome inhibitor. To put it differently, in accordance with this embodiment, the treatment comprises administering a therapeutically effective amount of a compound of Formula (I) above or a pharmaceutically acceptable salt thereof to a cancer patient who is under treatment of a proteasome inhibitor, or administering a therapeutically effective amount of a proteasome inhibitor to a cancer patient who is under treatment of a compound of Formula (I) above or a pharmaceutically acceptable salt thereof.

In another embodiment, the present invention provides a use of a compound of Formula (I) above or a pharmaceutically acceptable salt thereof for the manufacture of a medicament useful in combination with a proteasome inhibitor such as bortezomib for treating cancer. In yet another embodiment, the present invention provides a use of a proteasome inhibitor such as bortezomib for the manufacture of a medicament useful in combination with a compound of Formula (I) above or a pharmaceutically acceptable salt thereof for treating cancer.

In preferred embodiments, the compound is tris(8-quinolinolato)gallium(III) 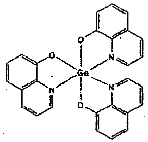 or a pharmaceutically acceptable salt thereof.

Proteasome inhibitors are inhibitors of the 26S proteasome in mammalian cells. The 26S proteasome is a large protein complex that degrades ubiquitinated proteins. The ubiquitin-proteasome pathway plays an essential role in regulating the intracellular concentration of specific proteins, thereby maintaining homeostasis within cells. Inhibition of the 26S proteasome prevents this targeted proteolysis, which can affect multiple signaling cascades within the cell including the NF-κB pathway. This disruption of normal homeostatic mechanisms can lead to cell death. Many proteasome inhibitors specifically inhibit the chymotrypsin-like activity of the 26S proteasome in mammalian cells. Any known proteasome inhibitors known in the art are useful in the present invention, including, but not limited to, those disclosed in U.S. Pat. Nos. 5,780,454 and 7,442,830; WO 07/0005991, WO 02/096933, WO 05/016859, WO 05/021558 and WO 06/08660, the relevant part of each of which is incorporated herein by reference.

Preferred proteasome inhibitors for the present invention may be selected from the group consisting of peptidyl aldehydes, boronic acids, boronic esters, lactacystin, and lactacystin analogs. In preferred embodiments, the proteasome inhibitor may be lactacystin or a lactacystin analog, particularly lactacystin, *clasto*-lactacystin ß-lactone, 7-ethyl-*clasto-*lactacystin β-lactone, 7-n-propyl-*clasto*-lactacystin β-lactone, or 7-n-butyl-*clasto*-lactacystin ß-lactone.

In some embodiments, the protéasome inhibitor is selected from the group of [(1R)-1-({[(2,3-difluorobenzoyl)amino]acetyl}amino)-3-methylbutyl]boronic acid, [(1R)-1-({[(5-chloro-2-fluorobenzoyl)amino]acetyl}amino)-3-methylbutyl]boronic acid, [(1R)-1-({[(3,5-difluorobenzoyl)amino]acetyl}amino)-3-methylbutyl]boronic acid, [(1R)-1-({[(2,5-difluorobenzoyl)amino]acetyl}amino)-3-methylbutyl]boronic acid, [(1R)-1-({[(2-bromobenzoyl)amino]acetyl}amino)-3-methylbutyl]boronic acid, [(1R)-1-({[(2-fluorobenzoyl)amino]acetyl}amino)-3-methylbutyl]boronic acid, [(1R)-1-({[(2-chloro-5-fluorobenzoyl)amino]acetyl}amino)-3-methylbutyl]boronic acid, [(1R)-1-({[(4-fluorobenzoyl)amino]acetyl}amino)-3-methylbutyl]boronic acid, [(1R)-1-({[(3,4-difluorobenzoyl)amino]acetyl}amino)-3-methylbutyl]boronic acid, [(1R)-1-({[(3-chlorobenzoyl)amino]acetyl}amino)-3-methylbutyl]boronic acid, [(1R)-1-({[(2,5-dichlorobenzoyl)amino]acetyl}amino)-3-methylbutyl]boronic acid, [(1R)-1-({[(3,4-dichlorobenzoyl)amino]acetyl}amino)-3-methylbutyl]boronic acid, [(1R)-1-({[(3-fluorobenzoyl)amino]acetyl}amino)-3-methylbutyl]boronic acid, [(1R)-1-({[(2-chloro-4-fluorobenzoyl)amino]acetyl}amino)-3-methylbutyl]boronic acid, [(1R)-1-({[(2,3-dichlorobenzoyl)amino]acetyl}amino)-3-methylbutyl]boronic acid, [(1R)-1-({[(2-chlorobenzoyl)amino]acetyl}amino)-3-methylbutyl]boronic acid, [(1R)-1-({[(2,4-difluorobenzoyl)amino]acetyl}amino)-3-methylbutyl]boronic acid, [(1R)-1-({[(4-chloro-2-fluorobenzoyl)amino]acetyl}amino) -3-methylbutyl]boronic acid, [(1R)-1-({[(4-chlorobenzoyl)amino]acetyl}amino)-3-methylbutyl]boronic acid, [(1R)-1-({[(2,4-dichlorobenzoyl)amino]acetyl}amino)-3-methylbutyl]boronic acid, and [(1R)-1-({[(3,5-dichlorobenzoyl)amino]acetyl}amino)-3-methyl.butyl]boronic acid, and esters (e.g., mannitol esters), salts thereof and boronic acid anhydrides thereof.

In preferred embodiments, the proteasome inhibitor is bortezomib, CEP-18770 (*See* Piva et al., Blood, 111(5):2765-75 (2008)), carfilzomib or MLN9708.

In some preferred embodiments, the combination of the present invention is used in treating hematological neoplasms such as multiple myeloma, lymphoma (e.g., multiple lymphoma, mantle cell lymphoma, Hodgkin lymphoma, non-Hodgkin's lymphoma, diffuse large cell lymphoma, lymphoplasmacytic lymphoma, Mature T cell and natural killer (NK) cell neoplasms), and leukemia (e.g., acute myelogenous leukemia, myelodysplasia, and myelodysplastic syndromes). In other embodiments, the combination is used in treating amyloidosis, chronic graft versus host disease, and in preventing rejection in transplantation.

In a specific embodiment, multiple myeloma is treated by simultaneously or sequentially administering a pharmaceutically effective amount of (1) tris(8-quinolinolato)gallium(III) and (2) a proteasome inhibitor (e.g., bortezomib, CEP-18770, carfilzomib or MLN9708, preferably bortezomib).

In other embodiments, the combination of the present invention is used in treating solid tumors particularly lung cancer, colon cancer, breast cancer, ovarian cancer, prostate cancer, liver cancer, mesothelioma, urethral or bladder cancer, renal cancer, neuroblastoma, glioblastoma, gliosarcoma, and melanoma.

In certain embodiments, the combination therapy is used to treat cancer patients having tumors that exhibit resistance to bortezomib or another proteasome inhibitor. In other words, the combination of the present invention is used to treat a cancer patient having previously been treated with a proteasome inhibitor such as bortezomib, and whose cancer was found to be non-responsive to the proteasome inhibitor or have developed resistance to the proteasome inhibitor treatment. In other embodiments, the combination of the present invention is used to treat a cancer patient refractory to a treatment with a proteasome inhibitor such as bortezomib, that is, who has previously been treated with a proteasome inhibitor such as bortezomib, and whose cancer was initially responsive to the previously administered proteasome inhibitor such as bortezomib, but was subsequently found to have relapsed.

Patients undergoing initial treatment with a proteasome inhibitor such as bortezomib can be carefully monitored for signs of resistance, non-responsiveness or refractory cancer. This can be accomplished by monitoring the patient's cancer's response to the proteasome inhibitor such as bortezomib treatment. The response, lack of response, or relapse of the cancer to initial treatment with a proteasome inhibitor such as bortezomib can be determined by any suitable method practiced in the art. For example, in the case of solid tumor cancers this can be accomplished by the assessment of tumor size and number. An increase in tumor size or, alternatively, tumor number, indicates that the tumor is not responding to the chemotherapy, or that a relapse has occurred. The determination can be done according to the "RECIST" criteria as described in detail in Therasse et al, J. Natl. Cancer Inst. 92:205-216(2000).

Thus, in these various embodiments, in accordance with the present invention, a patient having cancer (e.g., the specific types of cancer described above) is identified or diagnosed, and such patient is treated with a therapeutically effective amount of a proteasome inhibitor such as bortezomib as well as a therapeutically effective amount of a compound of Formula (I) such as tris(8-quinolinolato)gallium(III).

In the combination therapy of the present invention, the compound of Formula (I) such as tris(8-quinolinolato)gallium(III) and the proteasome inhibitor can be administered at about the same time, or separately according to their respective dosing schedules. When administered at about the same time, the gallium compound and the proteasome inhibitor can be administered in the same pharmaceutical composition or in separate dosage unit forms. For example, in one embodiment of the combination therapy of the present invention, the compound tris(8-quinolinolato)gallium(III) can be administered, e.g., orally at a dosing of from 0.1 mg to 3000 mg at, e.g., four times a day, while the proteasome inhibitor bortezomib may be administered intravenously (e.g., bolus IV injection) once or twice weekly each at an amount of from 0.7 mg/m² to about 1.3 mg/m².

It should be understood that the dosage ranges set forth above are exemplary only and are not intended to limit the scope of this invention. The therapeutically effective amount for each active compound can vary with factors including but not limited to the activity of the compound used, stability of the active compound in the patient's body, the severity of the conditions to be alleviated, the total weight of the patient treated, the route of administration, the ease of absorption, distribution, and excretion of the active compound by the body, the age and sensitivity of the patient to be treated, adverse events, and the like, as will be apparent to a skilled artisan. The amount of administration can be adjusted as the various factors change over time.

The pharmaceutical compound in the combination therapy of present invention can be administered in any suitable unit dosage forms. Suitable oral formulations can be in the form of tablets, caspules, suspension, syrup, chewing gum, wafer, elixir, and the like. Pharmaceutically acceptable carriers such as binders, excipients, lubricants, and sweetening or flavoring agents can be included in the oral pharmaceutical compositions. If desired, conventional agents for modifying tastes, colors, and shapes of the special forms can also be included. In addition, for convenient administration by enteral feeding tube in patients unable to swallow, the active compounds can be dissolved in an acceptable lipophilic vegetable oil vehicle such as olive oil, corn oil and safflower oil.

For injectable formulations, the pharmaceutical compositions can be in lyophilized powder in admixture with suitable excipients in a suitable vial or tube. Before use in the clinic, the drugs may be reconstituted by dissolving the lyophilized powder in a suitable solvent system to form a composition suitable for intravenous or intramuscular injection.

In accordance with another aspect of the present invention, a pharmaceutical composition is provided, comprising a therapeutically effective amount of a compound of Formula (I) such as tris(8-quinolinolato)gallium(III) as well as a therapeutically effective amount of a proteasome inhibitor.

In accordance with another aspect of the present invention, a pharmaceutical kit is provided comprising, in a compartmentalized container, (1) a unit dosage form of a compound of Formula (I) such as tris(8-quinolinolato)gallium(III); and (2) a unit dosage form of a proteasome inhibitor. As will be apparent to a skilled artisan, the amount of a therapeutic compound in the unit dosage form is determined by the dosage to be used on a patient in accordance with the present invention. In one embodiment of the kit, tris(8-quinolinolato)gallium(III) is in a tablet or capsule or any other suitable form at an amount of, e.g., 0.1 mg to about 3000 mg per unit dosage form. The kit further includes a proteasome inhibitor in a unit dosage of from about 0.1 mg to about 2000 mg in a tablet or capsule form or in lyophilized powder. In a specific embodiment, the proteasome inhibitor is bortezomib, and the kit includes a vial containing from about 0.5 mg to about 10 mg, preferably about 3.5 mg of bortezomib as a sterile lyophilized powder. Optionally, the kit further comprises instructions for using the kit in the combination therapy in accordance with the present invention.

### EXAMPLE 1

To determine whether the combination of tris(8-quinolinolato)gallium(III) and bortezomib produces synergistic effect using the constant ratio combination design of Chou and Talalay, the combination was tested in cell lines including multiple myeloma cell lines ARH-77, RPMI 8226, OPM-2, NCI-H929 with Promega's Cell Titer-Glo^{®} assay. Specifically, the human tumor cells were placed in a 96-well microculture plate at the appropriate density for 96 hours of total growth time. After 24 hours of incubation in a humidified incubator at 37 °C with 5% CO₂ and 95% air, serially diluted test agents in growth medium were added to each well. After 96 total hours of culture in a CO₂ incubator, the plates were processed with Cell Titer-Glo (Promega #G7571) according to manufacturer's instructions. Luminescence was detected using a Tecan GENios microplate reader. Percent inhibition of cell growth was calculated relative to untreated control wells. All tests were performed in duplicate at each concentration level.

The cells (in quadruplicate wells) were incubated with tris(8-quinolinolato)gallium(III) alone, bortezomib alone, or mixture of tris(8-quinolinolato)gallium(III) and bortezomib. Drug ratios were equivalent to the ratio of respective IC₅₀ of agents being combined. Assuming a combination response of near additivity, drug concentrations bracketed the sum of one half of the respective IC₅₀'s with serial dilutions selected based upon the inhibition curves of the agents being combined, (typically 1.5 fold dilutions) with a total of seven drug concentrations.

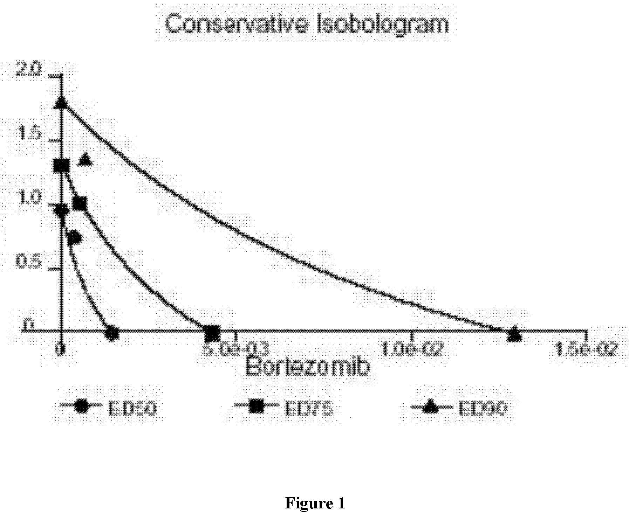

Combination of tris(8-quinolinolato)gallium(III) and bortezomib showed synergism. For example, Figure 1 is a conservative isobologram showing the synergism created by the combination of tris(8-quinolinolato)gallium(III) and bortezomib in ARH-77 cells. Y axis represents the amount of tris(8-quinolinolato)gallium(III) in µM and X axis represents the amount of bortezomib in µM.

### EXAMPLE 2

MTT cytotoxicity assays were performed with combination of tris(8-quinolinolato)gallium(III) and bortezomib in lung cancer cell lines A549 and A427, and colon cancer cell line SW480.

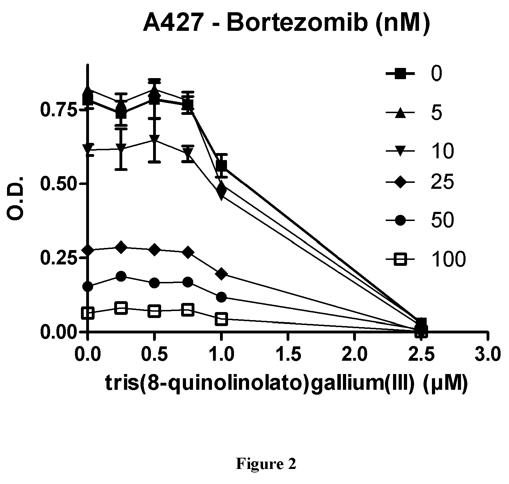

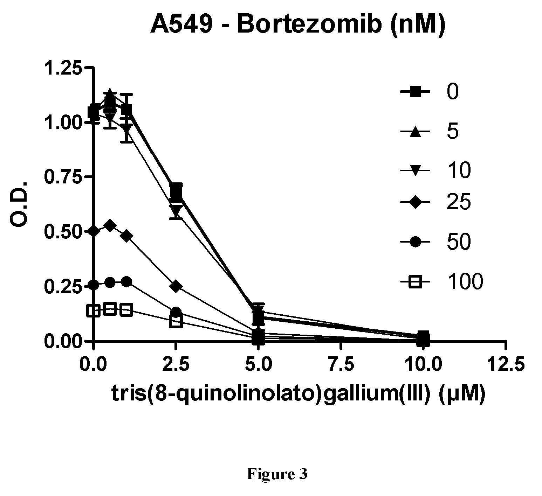

Cells were plated (2x103 cells in 100 µl/well) in 96-well plates and allowed to recover for 24 hours before drugs were added in another 100 µl growth medium and cells exposed for the indicated time periods. For pulsing experiments, test medium was replaced with fresh (drug-free) culture medium after the indicated exposure times. After 72 hours of drug treatment, the proportion of viable cells was determined after by MTT assay following the manufacturer's recommendations (EZ4U, Biomedica, Vienna, Austria). Combination of tris(8-quinolinolato)gallium(III) and bortezomib showed synergism in cells. For example, Figures 2 and 3 are graphs showing synergism between tris(8-quinolinolato)gallium(III) and bortezomib in A427 and A549 cells, respectively.

All publications and patent applications mentioned in the specification are indicative of the level of those skilled in the art to which this invention pertains.

Although the foregoing invention has been described in some detail by way of illustration and example for purposes of clarity of understanding, it will be apparent that certain changes and modifications may be practiced within the scope of the appended claims.

## Claims

1. Use of a therapeutically effective amount of a compound of Formula (I) 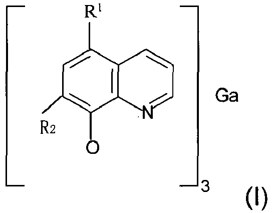 wherein R¹ represents hydrogen, a halogen or a sulfono group SO₃M, in which M is a metal ion, and R² represents hydrogen, or R¹ is Cl and R² is I, or a pharmaceutically acceptable salt thereof for the manufacture of a medicament for use in combination with a proteasome inhibitor for treating a proliferative disorder.

2. A compound of Formula (I) 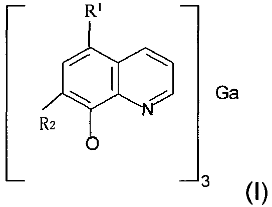 wherein R¹ represents hydrogen, a halogen or a sulfono group SO₃M, in which M is a metal ion, and R² represents hydrogen, or R¹ is Cl and R² is I, or a pharmaceutically acceptable salt thereof for use in treating a proliferative disorder, wherein said compound is to be used in combination with a proteasome inhibitor.

3. A proteasome inhibitor for use in treating a proliferative disorder, wherein said proteasome inhibitor is to be used in combination with a compound of Formula (I) 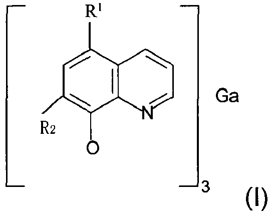 wherein R¹ represents hydrogen, a halogen or a sulfono group SO₃M, in which M is a metal ion, and R² represents hydrogen, or R¹ is Cl and R² is I, or a pharmaceutically acceptable salt thereof.

4. The use of Claim 1 or the compound for use according to Claim 2 or the proteasome inhibitor for use according to Claim 3, wherein said compound of Formula (I) is tris(8-quinolinolato)gallium(III).

5. The use of Claim 1 or 4 or the compound for use according to Claim 2 or 4 or the proteasome inhibitor for use according to Claim 3 or 4, wherein the proteasome inhibitor is bortezomib.

6. The use of Claim 1 or 4 or the compound for use according to Claim 2 or 4 or the proteasome inhibitor for use according to Claim 3 or 4, wherein the proteasome inhibitor is chosen from the group of [(1R)-1-({[(2,3-difluorobenzoyl)amino]acetyl}amino)-3-methylbutyl]boronic acid, [(1R)-1-({[(5-chloro-2-fluorobenzoyl)amino]acetyl}amino)-3-methylbutyl]boronic acid, [(1R)-1-({[(3,5-difluorobenzoyl)amino]acetyl}amino)-3-methylbutyl]boronic acid, [(1R)-1-({[(2,5-difluorobenzoyl)amino]acetyl}amino)-3-methylbutyl]boronic acid, [(1R)-1-({[(2-bromobenzoyl)amino]acetyl}amino)-3-methylbutyl]boronic acid, [(1R)-1-({[(2-fluorobenzoyl)amino]acetyl}amino)-3-methylbutyl]boronic acid, [(1R)-1-({[(2-chloro-5-fluorobenzoyl)amino]acetyl}amino)-3-methylbutyl]boronic acid, [(1R)-1-({[(4-fluorobenzoyl)amino]acetyl}amino)-3-methylbutyl]boronic acid, [(1R)-1-({[(3,4-difluorobenzoyl)amino]acetyl}amino)-3-methylbutyl]boronic acid, [(1R)-1-({[(3-chlorobenzoyl)amino]acetyl}amino)-3-methylbutyl]boronic acid, [(1 R)-1-({[(2,5-dichlorobenzoyl)amino]acetyl}amino)-3-methylbutyl]boronic acid, [(1R)-1-({[(3,4-dichlorobenzoyl)amino]acetyl}amino)-3-methylbutyl]boronic acid, [(1R)-1-({[(3-fluorobenzoyl)amino]acetyl}amino)-3-methylbutyl]boronic acid, [(1R)-1-({[(2-chloro-4-fluorobenzoyl)amino]acetyl}amino) -3-methylbutyl]boronic acid, [(1R)-1-({[(2,3-dichlorobenzoyl)amino]acetyl}amino)-3-methylbutyl]boronic acid, [(1R)-1-({[(2-chlorobenzoyl)amino]acetyl}amino)-3-methylbutyl]boronic acid, [(1R)-1-({[(2,4-difluorobenzoyl)amino]acetyl}amino)-3-methylbutyl]boronic acid, [(1R)-1-({[(4-chloro-2-fluorobenzoyl)amino]acetyl}amino)-3-methylbutyl]boronic acid, [(1R)-1-({[(4-chlorobenzoyl)amino]acetyl}amino)-3-methylbutyl]boronic acid, [(1R)-1-({[(2,4-dichlorobenzoyl)amino]acetyl}amino)-3-methylbutyl]boronic acid, and [(1R)-1-({[(3,5-dichlorobenzoyl)amino]acetyl}amino)-3-methylbutyl]boronic acid, and mannitol esters, salts and boronic acid anhydrides thereof.

7. The use of Claim 1 or 4 or the compound for use according to Claim 2 or 4 or the proteasome inhibitor for use according to Claim 3 or 4, wherein the proteasome inhibitor is CEP-18770, carfilzomib or MLN9708.

8. The use of any one of Claims 1 and 4 to 7 or the compound for use according to any one of Claims 2 and 4 to 7 or the proteasome inhibitor for use according to any one of Claims 3 to 7, wherein the proliferative disorder is cancer.

9. The use of any one of Claims 1 and 4 to 7 or the compound for use according to any one of Claims 2 and 4 to 7 or the proteasome inhibitor for use according to any one of Claims 3 to 7, wherein the proliferative disorder is multiple myeloma.

10. The use of any one of Claims 1 and 4 to 7 or the compound for use according to any one of Claims 2 and 4 to 7 or the proteasome inhibitor for use according to any one of Claims 3 to 7, wherein the proliferative disorder is a solid tumor.

11. The use of any one of Claims 1 and 4 to 7 or the compound for use according to any one of Claims 2 and 4 to 7 or the proteasome inhibitor for use according to any one of Claims 3 to 7, wherein the proliferative disorder is a hematological cancer.

12. A kit, comprising in a compartmentalized container:
a first unit dosage form having a compound of Formula (I) 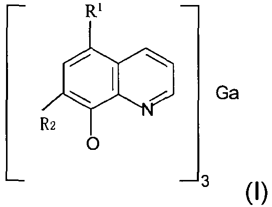 wherein R¹ represents hydrogen, a halogen or a sulfono group SO₃M, in which M is a metal ion, and R² represents hydrogen, or R¹ is Cl and R² is I, or a pharmaceutically acceptable salt thereof; and
a second unit dosage form having a proteasome inhibitor.

13. The kit of Claim 12, wherein the compound of Formula (I) is tris(8-quinolinolato)gallium(III).

14. A pharmaceutical composition, comprising a therapeutically effective amount of a compound of Formula (I) 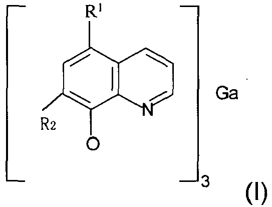 wherein R¹ represents hydrogen, a halogen or a sulfono group SO₃M, in which M is a metal ion, and R² represents hydrogen, or R¹ is Cl and R² is I, or a pharmaceutically acceptable salt thereof and a proteasome inhibitor.

15. The pharmaceutical composition of Claim 14, wherein said compound of Formula(l) is tris(8-quinolinolato)gallium(III).

## Patentansprüche

1. Verwendung einer therapeutisch wirksamen Menge einer Verbindung der
Formel (I) 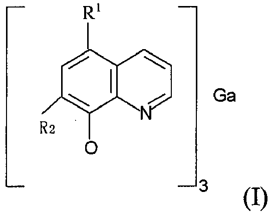 worin R¹ Wasserstoff, ein Halogen oder eine Sulfogruppe SO₃M ist, in welcher M ein Metallion ist und R² Wasserstoff darstellt, oder R¹ Cl ist und R² I ist, oder ein pharmazeutisch verträgliches Salz davon, für die Herstellung eines Medikaments zur Verwendung in Kombination mit einem Proteasom-Inhibitor zur Behandlung einer proliferativen Erkrankung.

2. Verbindung der Formel (I) 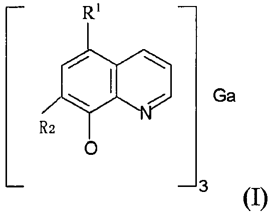 worin R¹ Wasserstoff, ein Halogen oder eine Sulfogruppe SO₃M ist, in welcher M ein Metallion ist und R² Wasserstoff darstellt, oder R¹ Cl ist und R² I ist, oder ein pharmazeutisch verträgliches Salz davon, zur Verwendung bei der Behandlung einer proliferativen Erkrankung, wobei besagte Verbindung in Kombination mit einem Proteasom-Inhibitor verwendet wird.

3. Proteasom-Inhibitor zur Verwendung bei der Behandlung einer proliferativen Erkrankung, wobei besagter Proteasom-Inhibitor in Verwendung mit einer Verbindung der Formel (I) 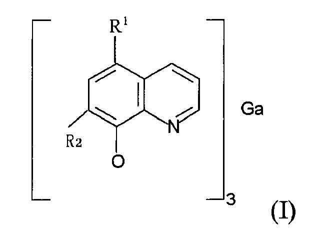 verwendet werden soll, wobei R¹ Wasserstoff, ein Halogen oder eine Sulfogruppe SO₃M ist, in welcher M ein Metallion ist und R² Wasserstoff darstellt, oder R¹ Cl ist und R² I ist, oder ein pharmazeutisch verträgliches Salz davon.

4. Verwendung nach Anspruch 1 oder Verbindung zur Verwendung nach Anspruch 2 oder Proteasom-Inhibitor zur Verwendung nach Anspruch 3, wobei besagte Verbindung der Formel (I) Tris(8-quinolinolato)gallium(III) ist.

5. Verwendung nach Anspruch 1 oder 4 oder Verbindung zur Verwendung nach Anspruch 2 oder 4 oder Proteasom-Inhibitor zur Verwendung nach Anspruch 3 oder 4, wobei der Proteasom-Inhibitor Bortezomib ist.

6. Verwendung nach Anspruch 1 oder 4 oder Verbindung zur Verwendung nach Anspruch 2 oder 4 oder Proteasom-Inhibitor zur Verwendung nach Anspruch 3 oder 4, wobei der Proteasom-Inhibitor ausgewählt ist aus der Gruppe bestehend aus: [(1R)-1-({[(2,3-difluorobenzoyl)amino]acetyl}amino)-3-methylbutyl]borsäure, [(1R)-1-({[(5-chloro-2-fluorobenzoyl)amino]acetyl}amino)-3-methylbutyl]borsäure, [(1R)-1-({[(3,5-difluorobenzoyl)amino]acetyl}amino)-3-methylbutyl]borsäure, [(1R)-1-({[(2,5-difluorobenzoyl)amino]acetyl}amino)-3-methylbutyl]borsäure, [(1R)-1-({[(2-bromobenzoyl)amino]acetyl}amino)-3-methylbutyl]borsäure, [(1R)-1-({[(2-fluorobenzoyl)amino]acetyl}amino)-3-methylbutyl]borsäure, [(1R)-1-({[(2-chloro-5-fluorobenzoyl)amino]acetyl}amino)-3-methylbutyl]borsäure, [(1R)-1-({[(4-fluorobenzoyl)amino]acetyl}amino)-3-methylbutyl]borsäure, [(1R)-1-({[(3,4-difluorobenzoyl)amino]acetyl}amino)-3-methylbutyl]borsäure, [(1R)-1-({[(3-chlorobenzoyl)amino]acetyl}amino)-3-methylbutyl]borsäure, [(1R)-1-({[(2,5-dichlorobenzoyl)amino]acetyl}amino)-3-methylbutyl]borsäure, [(1R)-1-({[(3,4-dichlorobenzoyl)amino]acetyl}amino)-3-methylbutyl]borsäure, [(1R)-1-({[(3-fluorobenzoyl)amino]acetyl}amino)-3-methylbutyl]borsäure, [(1R)-1-({[(2-chloro-4-fluorobenzoyl)amino]acetyl}amino)-3-methylbutyl]borsäure, [(1R)-1-({[(2,3-dichlorobenzoyl)amino]acetyl}amino)-3-methylbutyl]borsäure, [(1R)-1-({[(2-chlorobenzoyl)amino]acetyl}amino)-3-methylbutyl]borsäure, [(1R)-1-({[(2,4-difluorobenzoyl)amino]acetyl}amino)-3-methylbutyl]borsäure, [(1R)-1-({[(4-chloro-2-fluorobenzoyl)amino]acetyl}amino)-3-methylbutyl]borsäure, [(1R)-1-({[(4-chlorobenzoyl)amino]acetyl}amino)-3-methylbutyl]borsäure, [(1R)-1-({[(2,4-dichlorobenzoyl)amino]acetyl}amino)-3-methylbutyl]borsäure und [(1R)-1-({[(3,5-dichlorobenzoyl)amino]acetyl}amino)-3-methylbutyl]borsäure, und Mannitolester, Salze und Borsäureanhydride davon.

7. Verwendung nach Anspruch 1 oder 4 oder Verbindung zur Verwendung nach Anspruch 2 oder 4 oder Proteasom-Inhibitor zur Verwendung nach Anspruch 3 oder 4, wobei der Proteasom-Inhibitor CEP-18770, Carfilzomib oder MLN9708 ist.

8. Verwendung nach einem der Ansprüche 1 und 4 bis 7 oder Verbindung zur Verwendung nach einem der Ansprüche 2 und 4 bis 7 oder Proteasom-Inhibitor zur Verwendung nach einem der Ansprüche 3 bis 7, wobei die proliferative Erkrankung Krebs ist.

9. Verwendung nach einem der Ansprüche 1 und 4 bis 7 oder Verbindung zur Verwendung nach einem der Ansprüche 2 und 4 bis 7 oder Proteasom-Inhibitor zur Verwendung nach einem der Ansprüche 3 bis 7, wobei die proliferative Erkrankung ein multiples Myelom ist.

10. Verwendung nach einem der Ansprüche 1 und 4 bis 7 oder Verbindung zur Verwendung nach einem der Ansprüche 2 und 4 bis 7 oder Proteasom-Inhibitor zur Verwendung nach einem der Ansprüche 3 bis 7, wobei die proliferative Erkrankung ein fester Tumor ist.

11. Verwendung nach einem der Ansprüche 1 und 4 bis 7 oder Verbindung zur Verwendung nach einem der Ansprüche 2 und 4 bis 7 oder Proteasom-Inhibitor zur Verwendung nach einem der Ansprüche 3 bis 7, wobei die proliferative Erkrankung ein hämatologischer Krebs ist.

12. Kit, umfassend in einem Behälter mit Kammern:
eine erste Einheitsdosierungsform mit einer Verbindung der Formel (I) 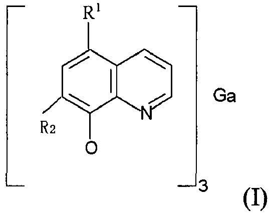 wobei R¹ Wasserstoff, ein Halogen oder eine Sulfogruppe SO₃M ist, in welcher M ein Metallion ist und R² Wasserstoff darstellt, oder R¹ Cl ist und R² I ist, oder ein pharmazeutisch verträgliches Salz davon; und
eine zweite Einheitsdosierungsform mit einem Proteasom-Inhibitor.

13. Kit nach Anspruch 12, wobei die Verbindung der Formel (I) Tris(8-quinolinolato)gallium(III) ist.

14. Pharmazeutische Zusammensetzung, umfassend eine therapeutisch wirksame Menge einer Verbindung der Formel (I) 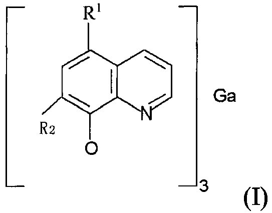 worin R¹ Wasserstoff, ein Halogen oder eine Sulfogruppe SO₃M ist, in welcher M ein Metallion ist und R² Wasserstoff darstellt, oder R¹ Cl ist und R² I ist, oder ein pharmazeutisch verträgliches Salz davon, und einen Proteasom-Inhibitor.

15. Pharmazeutische Zusammensetzung nach Anspruch 14, wobei besagte Verbindung der Formel (I) Tris(8-quinolinolato)gallium(III) ist.

## Revendications

1. Utilisation d'une quantité ayant une efficacité thérapeutique d'un composé de formule (I) 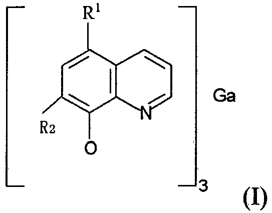 dans laquelle R¹ représente l'hydrogène, un halogène ou un groupe sulfono SO₃M, où M est un ion métallique, et R² représente l'hydrogène, ou bien R¹ est Cl et R² est I,
ou d'un sel pharmaceutiquement acceptable de celui-ci, pour la fabrication d'un médicament destiné à être utilisé en combinaison avec un inhibiteur de protéasome pour le traitement d'un trouble prolifératif.

2. Composé de formule (I) 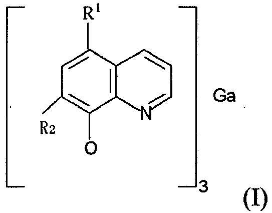 dans laquelle R¹ représente l'hydrogène, un halogène ou un groupe sulfono SO₃M, où M est un ion métallique, et R² représente l'hydrogène, ou bien R¹ est Cl et R² est I,
ou un sel pharmaceutiquement acceptable de celui-ci, pour utilisation dans le traitement d'un trouble prolifératif, lequel composé est destiné à être utilisé en combinaison avec un inhibiteur de protéasome.

3. Inhibiteur de protéasome pour utilisation dans le traitement d'un trouble prolifératif, lequel inhibiteur de protéasome est destiné à être utilisé en combinaison avec un composé de formule (I) 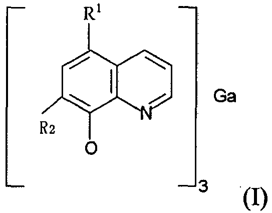 dans laquelle R¹ représente l'hydrogène, un halogène ou un groupe sulfono SO₃M, où M est un ion métallique, et R² représente l'hydrogène, ou bien R¹ est Cl et R² est I,
ou un sel pharmaceutiquement acceptable de celui-ci.

4. Utilisation selon la revendication 1 ou composé pour utilisation selon la revendication 2 ou inhibiteur de protéasome pour utilisation selon la revendication 3, dans lequel ledit composé de formule (I) est le tris(8-quinolinolato)gallium(III).

5. Utilisation selon la revendication 1 ou 4 ou composé pour utilisation selon la revendication 2 ou 4 ou inhibiteur de protéasome pour utilisation selon la revendication 3 ou 4, dans lequel l'inhibiteur de protéasome est le bortézomib.

6. Utilisation selon la revendication 1 ou 4 ou composé pour utilisation selon la revendication 2 ou 4 ou inhibiteur de protéasome pour utilisation selon la revendication 3 ou 4, dans lequel l'inhibiteur de protéasome est choisi dans le groupe constitué par l'acide [(1R)-1-({[(2,3-difluorobenzoyl)amino]acétyl}amino)-3-méthylbutyl]boronique, l'acide [(1R)-1-({[(5-chloro-2-fluorobenzoyl)amino]acétyl}amino)-3-méthylbutyl]boronique, l'acide [(1R)-1-({[(3,5-difluorobenzoyl)amino]acétyl}amino)-3-méthylbutyl]boronique, l'acide [(1R)-1-({[(2,5-difluorobenzoyl)amino]acétyl}amino)-3-méthylbutyl]boronique, l'acide [(1R)-1-({[(2-bromobenzoyl)amino]acétyl}amino)-3-méthylbutyl]boronique, l'acide [(1R)-1-({[(2-fluorobenzoyl)amino]acétyl}amino)-3-méthylbutyl]boronique, l'acide [(1R)-1-({[(2-chloro-5-fluorobenzoyl)amino]acétyl}amino)-3-méthylbutyl]boronique, l'acide [(1R)-1-({[(4-fluorobenzoyl)amino]acétyl}amino)-3-méthylbutyl]boronique, l'acide [(1R)-1-({[(3,4-difluorobenzoyl)amino]acétyl}amino)-3-méthylbutyl]boronique, l'acide [(1R)-1-({[(3-chlorobenzoyl)amino]acétyl}amino)-3-méthylbutyl]boronique, l'acide [(1R)-1-({[(2,5-dichlorobenzoyl)amino]acétyl}amino)-3-méthylbutyl]boronique, l'acide [(1R)-1-({[(3,4-dichlorobenzoyl)amino]acétyl}amino)-3-méthylbutyl]boronique, l'acide [(1R)-1-({[(3-fluorobenzoyl)amino]acétyl}amino)-3-méthylbutyl]boronique, l'acide [(1R)-1-({[(2-chloro-4-fluorobenzoyl)amino]acétyl}amino)-3-méthylbutyl]boronique, l'acide [(1R)-1-({[(2,3-dichlorobenzoyl)amino]acétyl}amino)-3-méthylbutyl]boronique, l'acide [(1R)-1-({[(2-chlorobenzoyl)amino]acétyl}amino)-3-méthylbutyl]boronique, l'acide [(1R)-1-({[(2,4-difluorobenzoyl)amino]acétyl}amino)-3-méthylbutyl]boronique, l'acide [(1R)-1-({[(4-chloro-2-fluorobenzoyl)amino]acétyl}amino)-3-méthylbutyl]boronique, l'acide [(1R)-1-({[(4-chlorobenzoyl)amino]acétyl}amino)-3-méthylbutyl]boronique, l'acide [(1R)-1-({[(2,4-dichlorobenzoyl)amino]acétyl}amino)-3-méthylbutyl]boronique, et l'acide [(1R)-1-({[(3,5-dichlorobenzoyl)amino]acétyl}amino)-3-méthylbutyl]boronique, ainsi que leurs esters de mannitol, leurs sels et leurs anhydrides d'acide boronique.

7. Utilisation selon la revendication 1 ou 4 ou composé pour utilisation selon la revendication 2 ou 4 ou inhibiteur de protéasome pour utilisation selon la revendication 3 ou 4, dans lequel l'inhibiteur de protéasome est CEP-18770, le carfilzomib ou MLN9708.

8. Utilisation selon l'une quelconque des revendications 1 et 4 à 7 ou composé pour utilisation selon l'une quelconque des revendications 2 et 4 à 7 ou inhibiteur de protéasome pour utilisation selon l'une quelconque des revendications 3 à 7, dans lequel le trouble prolifératif est un cancer.

9. Utilisation selon l'une quelconque des revendications 1 et 4 à 7 ou composé pour utilisation selon l'une quelconque des revendications 2 et 4 à 7 ou inhibiteur de protéasome pour utilisation selon l'une quelconque des revendications 3 à 7, dans lequel le trouble prolifératif est un myélome multiple.

10. Utilisation selon l'une quelconque des revendications 1 et 4 à 7 ou composé pour utilisation selon l'une quelconque des revendications 2 et 4 à 7 ou inhibiteur de protéasome pour utilisation selon l'une quelconque des revendications 3 à 7, dans lequel le trouble prolifératif est une tumeur solide.

11. Utilisation selon l'une quelconque des revendications 1 et 4 à 7 ou composé pour utilisation selon l'une quelconque des revendications 2 et 4 à 7 ou inhibiteur de protéasome pour utilisation selon l'une quelconque des revendications 3 à 7, dans lequel le trouble prolifératif est un cancer hématologique.

12. Trousse comprenant, dans un récipient compartimenté :
une première forme posologique unitaire ayant un composé de formule (I) 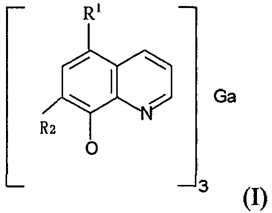 dans laquelle R¹ représente l'hydrogène, un halogène ou un groupe sulfono SO₃M, où M est un ion métallique, et R² représente l'hydrogène, ou bien R¹ est Cl et R² est I,
ou d'un sel pharmaceutiquement acceptable de celui-ci ; et
une deuxième forme posologique unitaire ayant un inhibiteur de protéasome.

13. Trousse selon la revendication 12, dans laquelle le composé de formule (I) est le tris(8-quinolinolato)gallium(III).

14. Composition pharmaceutique comprenant une quantité ayant une efficacité thérapeutique d'un composé de formule (I) 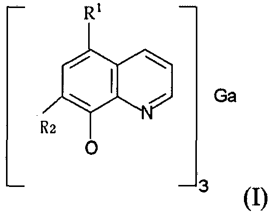 dans laquelle R¹ représente l'hydrogène, un halogène ou un groupe sulfono SO₃M, où M est un ion métallique, et R² représente l'hydrogène, ou bien R¹ est Cl et R² est I,
ou d'un sel pharmaceutiquement acceptable de celui-ci, et un inhibiteur de protéasome.

15. Composition pharmaceutique selon la revendication 14, dans laquelle le composé de formule (I) est le tris(8-quinolinolato)gallium(III).
